# EUROPÄISCHE PATENTSCHRIFT

(11) **EP 2 785 686 B1**
(45) Veröffentlichungstag und Bekanntmachung des Hinweises auf die Patenterteilung: **18.10.2017**
(21) Anmeldenummer: 12791486.9
(22) Anmeldetag: 28.11.2012
(51) Int. Cl.: C07C 303/44, C07C 309/15

(54) **VERFAHREN ZUR HERSTELLUNG UND AUFREINIGUNG VON SALZEN DER ACRYLAMIDO-2-METHYL-PROPANSULFONSÄURE**
METHOD FOR PRODUCING AND PURIFYING SALTS OF ACRYLAMIDO-2-METHYLPROPANE SULFONIC ACID
PROCÉDÉ DE PRODUCTION ET DE PURIFICATION DE SELS D'ACIDE ACRYLAMIDO-2-MÉTHYLPROPANSULFONIQUE

(30) Priorität: 29.11.2011 EP 11191117
(43) Veröffentlichungstag der Anmeldung: 08.10.2014
(73) Patentinhaber: BASF SE, 67056 Ludwigshafen am Rhein (DE)
(72) Erfinder: EBEL, Klaus, 68542 Heddesheim (DE); VOITL, Tobias, 67105 Schifferstadt (DE); KELLER, Andreas, 67346 Speyer (DE); RUEDENAUER, Stefan, 67549 Worms (DE); BARTLING, Karsten, 67433 Neustadt (DE); LANGLOTZ, Bjoern, 83308 Trostberg (DE); STEINER, Jochen, 64625 Bensheim (DE)
(86) Internationale Anmeldenummer: PCT/EP2012/073791
(87) Internationale Veröffentlichungsnummer: WO 2013/079507

(56) Entgegenhaltungen:
- WO-A2-2011/025847
- RO-B1- 104 626
- US-A- 4 337 215
- US-A- 4 650 614
- US-A1- 2010 274 048
- US-B1- 6 331 647
- DATABASE CA [Online] CHEMICAL ABSTRACTS SERVICE, COLUMBUS, OHIO, US; KAWAKAMI, TSUNEHIRO ET AL: "Purification of 2-acrylamido-2-methylpropanesulfonic acid", XP002698776, gefunden im STN Database accession no. 1991:608826 & JP 3 077860 A (NISSEI KAGAKU KOGYO K. K., JAPAN) 3. April 1991 (1991-04-03)
- DATABASE WPI Week 200441 Thomson Scientific, London, GB; AN 2004-434131 XP002698777, & JP 2004 143078 A (TOA GOSEI CHEM IND LTD) 20. Mai 2004 (2004-05-20)
- V.F. KURENKOV ET AL: "Copolymerization of acrylamide with salts of 2-acrylamido-2-methylpropanesulfonic acid in aqueous solutions as influenced by the nature of double-charged cation", JOURNAL OF APPLIED CHEMISTRY OF USSR., Bd. 74, Nr. 5, 2001, Seiten 813-817, XP002698778, CONSULTANTS BUREAU, NEW YORK, NY. ISSN: 0021-888X

## Beschreibung

Die Erfindung betrifft ein Verfahren zur Herstellung von Nebenprodukt-freien bzw. Nebenprodukt-armen Salzen von Acrylamido-2-methyl-propansulfonsäure (nachfolgend ®AMPS oder Verbindung A). Die Erfindung betrifft insbesondere die Herstellung des Natriumsalzes der Verbindung A mit einer Reinheit von mindestens 99 %, insbesondere mindestens 99,5 %.

Bislang wurden gereinigte Salze der Verbindung (A) unter Verwendung der bereits vorab gereinigten Acrylamido-2-methyl-propansulfonsäure hergestellt, was jedoch zu Nachteilen führt. In der Literatur werden zahlreiche Verfahren zur Herstellung und zur Aufarbeitung der erhaltenen Verbindung (A) beschrieben. Auch zur Herstellung der Salze sind verschiedene Verfahrenswege bekannt.

Ein einfaches Herstellungsverfahren für Verbindung (A) lässt sich durch das folgende Reaktionsschema beschreiben, bei dem Acrylnitril im Überschuss als Lösemittel und Reaktand mit Isobuten und Schwefelsäure zur Reaktion gebracht wird. Dabei kann die verwendete Schwefelsäure auch verschieden große Anteile an freiem SO₃ enthalten. In einer Ausführungsform kann die Zugabe von SO₃ und Wasser auch getrennt erfolgen.

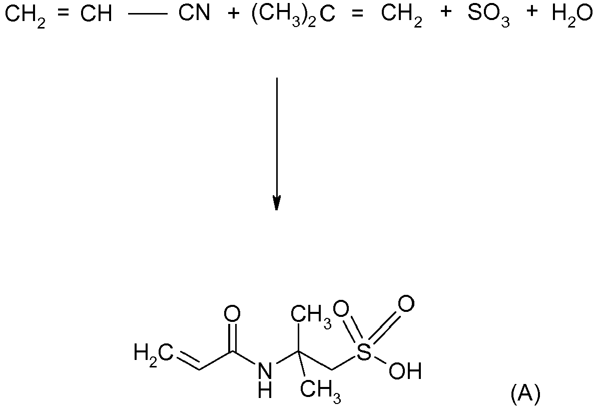

Bei einer Ausführungsform des Herstellungsverfahrens wird in einem kontinuierlichen Prozess zunächst Acrylnitril eingespeist und dann mit Isobuten und Oleum versetzt. Es ist aber auch die Durchführung eines diskontinuierlichen Verfahrens (Batch-Fahrweise) möglich.

Die Verbindung (A) ist ein farbloser, kristalliner Feststoff, der in Acrylnitril nur sehr schlecht löslich ist. Für die weitere Verarbeitung kommt der Reinheit der Verbindung (A) bzw. deren Salze auch deshalb besondere Bedeutung zu, da bei der Herstellung von Polymeren und Copolymeren aus Verbindung (A) bzw. deren Salze Verunreinigungen sehr nachteilige Eigenschaften mit sich bringen können. Insbesondere betrifft dies die Verwendung von Verbindung (A) als Monomer zur Herstellung von hochmolekularen Polymeren und Copolymeren, wie sie beispielsweise in der Erdölförderung eingesetzt werden, jedoch auch als Flockungsmittel, als Flüssigkeitsverlust-Polymere ("fluid loss polymers") und als Zementier-Polymere ("cementing polymers").

Das Patent US 4,337,215 beschreibt ein Reinigungsverfahren für die Herstellung von aufgereinigter 2-Acrylamido-2-methyl-propansulfonsäure. In dem Verfahren ist das Ausgangsmaterial ein roher, kristalliner Niederschlag von AMPS, der durch Waschen des Niederschlags aus dem in bekannter Weise hergestellten Reaktionsgemisch erhalten wurde. Die Rohkristalle werden in Essigsäure gelöst, die 5 bis 40% Wasser enthält. Die Menge an wässriger Essigsäure, die erforderlich ist, um die gewünschte Menge von Verbindung (A) bei 90°C vollständig zu lösen, hängt vom Wassergehalt ab. Weist die wässrige Essigsäure einen Wassergehalt von 10% auf, wird sie in 4- bis 5-facher Menge, bezogen auf das Gewicht der Rohkristalle verwendet. Die gereinigten Kristalle werden durch Filtration der Suspension bei 10 bis 20°C erhalten.

Aus US 4,701,283 sind Verfahren zur Herstellung von Verbindung (A) und deren Salzen bekannt, sowie Copolymer-beschichtete feste Materialien und Copolymer-Emulsionen, in denen das Copolymer durch Polymerisation der Verbindung (A) mit einem anderen Monomer hergestellt wird.

US 4,650,614 wird ein Verfahren zur Reinigung von technischer 2-Acrylamido-2-methylpropansulfonsäure beschrieben, die durch kurzzeitiges Erwärmen der Sulfonsäure in einer Aufschlämmung mit einem flüchtigen, einwertigen Alkohol und anschließender Rückgewinnung der Sulfonsäure durch Dekantieren oder einer anderen Form der Trennung und anschließendem Trocknen der feuchten, festen Sulfonsäure erhalten wird.

US 6,331,647 beschreibt die Herstellung und Aufreinigung von Acrylamidsulfonsäure-Monomeren.

Sie erfolgt durch Umsetzung einer verunreinigten Acrylamidsulfonsäure mit einer wässrigen Lösung von Metalloxiden oder -Hydroxiden, gefolgt von Kristallisation. Nachteilig an der Methode ist, dass aufgrund der hohen Löslichkeit der Zielprodukte in Wasser eine quantitative Abtrennung nur mit hohem Aufwand zu erreichen ist, und dass eine Aufreinigung der Zielprodukte erschwert ist, da einige der Verunreinigungen gleich gut oder sogar bevorzugt aus der Lösung kristallisieren. Hier insbesondere zu nennen sind die Sulfonsäuren 2-Methyl-2-propen-1-sulfonsäure (Isobutensulfonsäure, IBSA) und 2-Methyliden-1,3-propendisulfonsäure (Isobutendisulfonsäure, IBDSA). Somit werden bei der Polymerisation von Verbindung (A) oder des Natriumsalzes keine Polymere mit hohem Molekulargewicht erreicht. Als weitere Haupt-Nebenkomponente wird im Prozess tert-Butylacrylamid (ATB) erzeugt.

US 6,331,647 zeigt eine Aufreinigung des eingesetzten Salzes anhand der Reduktion der Peaks in einem HPLC-Chromatogramm. Dabei werden keine Sulfonsäuren erwähnt. Ebenfalls wird nicht auf die Anwendung des aufgereinigten Salzes für die Herstellung von Polymeren mit hohem Molekulargewicht eingegangen. Es wird nicht gezeigt, dass sich durch die Aufreinigung ein positiver Effekt in der Anwendung ergibt.

US-A 2010/274048 wird ein Verfahren zur Herstellung der Verbindung (A) beschrieben, bei dem ein Produkt erhalten wird, das weniger als 100 ppm an 2-Methyl-2-propenyl-1-sulfonsäure und weniger als 100 ppm an 2-Methyliden-1,3-propylen-disulfonsäure enthält. Die Aufreinigung erfolgt hierbei primär duch Kristallisation - nachfolgend wird der Gehalt der störenden Nebenkomponenten durch gezielte Wasch- und Trockenschritte bis auf den gewünschten Zielgehalt reduziert. Nachträglich kann das erhaltene (A) durch Umsetzung mit Basen analog zum Stand der Technik in das gewünschte Salz umgewandelt werden. Nachteilig an diesem Verfahren ist auch der aufwändige und Investitionskosten-intensive Aufreinigungs-Ablauf.

Eine Aufgabe der vorliegenden Erfindung ist es, ein einfaches und verbessertes Verfahren zur Herstellung von Salzen von Verbindung (A) mit hoher Reinheit bereitzustellen. Insbesondere soll der Gehalt an organischen Verunreinigungen minimiert werden, die bei der Polymerisation störenden Einfluss haben.

Diese Salze der Acrylamido-2-methyl-propansulfonsäure sind als Monomere zur Herstellung von Homopolymeren und Copolymeren mit sehr hohem Molekulargewicht von Bedeutung. Verschiedene Nebenprodukte der Herstellung von Verbindung (A) können bei der Polymerisation zu unerwünschten Produkten führen.

Diese Aufgabe wird gelöst durch ein Verfahren zur Herstellung von Salzen von Acrylamido-2-methyl-propansulfonsäure (A) umfassend die Schritte:
a) Herstellen einer Lösung eines verunreinigten Salzes der Acrylamido-2-methyl-propansulfonsäure (A) in einem Wasser-freien, organischen Lösungsmittel (L) unter Einsatz mindestens einer basischen Komponente (B) ausgewählt aus der Gruppe Alkalimetalloxide, Erdalkalimetalloxide, Alkalimetallhydroxide, Erdalkalimetallhydroxide und Amine der allgemeinen Formel (I)

   NR^{a} R^{b} R^{c} (I)

   wobei die Reste R^{a}, R^{b} und R^{c} unabhängig voneinander bedeuten:
   Wasserstoff, C₁-C₄-Alkyl, Hydroxy-C₁-C₄-alkyl oder C₁-C₄-Alkoxy,
   wobei das Molverhältnis von Verbindung (A) zur basischen Komponente (B) vorzugsweise bei 1:1 bis 1:3 liegt,
b) gegebenenfalls teilweises Entfernen des organischen Lösungsmittels (L) bei einem Druck im Bereich von 0,1 bis 200 kPa (abs),
c) Rückgewinnung des gelösten Salzes der Verbindung (A) durch Kristallisation oder durch Fällung, durch Veränderung der Temperatur und/oder des Drucks und/oder der Konzentration des Salzes in der Lösung,
d) gegebenenfalls Trocknung des gereinigten Salzes der Acrylamido-2-methyl-propansulfonsäure (A).

Die Erfindung betrifft auch ein Verfahren, dadurch gekennzeichnet, dass als wasserfreies Lösungsmittel (L) ein Lösungsmittel aus der Gruppe: Methanol, Ethanol, Propanol, Butanol, Acetonitril, Aceton, DMF, oder eine Mischung mindestens zweier dieser Lösungsmittel eingesetzt wird.

Die Erfindung betrifft auch ein Verfahren, dadurch gekennzeichnet, dass in Schritt a) ein Alkalimetall-Salz der Acrylamido-2-methyl-propansulfonsäure, insbesondere das Natrium-Salz eingesetzt wird.

Die Erfindung betrifft auch ein Verfahren, dadurch gekennzeichnet, dass in Schritt a) ein Salz der Acrylamido-2-methyl-propansulfonsäure (A) mit einem Amin der Formel (I), insbesondere ein Trimethylammonium-Salz eingesetzt wird.

Die Erfindung betrifft auch ein Verfahren, dadurch gekennzeichnet, dass in Schritt b) mindestens 50 Gew.% des organischen Lösungsmittels (L) bei einem Druck im Bereich von 0,1 bis 50 kPa (abs) entfernt werden.

Die Erfindung betrifft auch ein Verfahren, dadurch gekennzeichnet, dass in Schritt b) mindestens 60 Gew.% des organischen Lösungsmittels (L) unter Einspeisung eines Gases, insbesondere von Luft, entfernt werden.

Die Erfindung betrifft auch ein Verfahren, dadurch gekennzeichnet, dass in Schritt a) als Lösungsmittel (L) ein wasserfreier C₁-C₃-Alkohol verwendet wird und in Schritt c) das gereinigte Salz durch Temperaturveränderungen aus einer alkoholischen Lösung gewonnen wird.

Die Erfindung betrifft auch ein Verfahren, dadurch gekennzeichnet, dass in Schritt c) das gereinigte Salz durch Druckveränderungen aus der organischen Lösung gewonnen wird.

Die Erfindung betrifft auch ein Verfahren, dadurch gekennzeichnet, dass in Schritt c) das gereinigte Salz durch Konzentrationsveränderung und/oder durch Zusatz einer weiteren organischen Komponente (NL) aus der organischen Lösung gewonnen wird.

Die Erfindung betrifft auch ein Verfahren, dadurch gekennzeichnet, dass mindestens die Schritte a) und c) mehrfach wiederholt werden. Diese Schritte (Lösen mit wasserfreier Base, ggf. Entfernen des organischen Lösemittels und Rückgewinnung des Salzes) können z. B. 2 bis 10 Mal, insbesondere 2 bis 5 mal wiederholt werden, wodurch jeweils ein höhere Reinheit erzielt werden kann.

Eine Acrylamido-2-methyl-propansulfonsäure (A), bzw. deren Salz, ist herstellbar bzw. hergestellt nach einem Verfahren wie beschrieben. Aus dem Salz kann die freie Verbindung (A) hergestellt werden.

Auch eine Acrylamido-2-methyl-propansulfonsäure (A) bzw. ein Salz mit einer Reinheit von mindestens 99,5 %, insbesondere mit einer Reinheit von mindestens 99,8 %, wird beschrieben. Bevorzugt werden Natriumsalze mit einer Reinheit von mindestens 99,9 % beschrieben.

Die Acrylamido-2-methyl-propansulfonsäure (A), hergestellt nach einem Verfahren wie beschrieben, kann zur Herstellung von Copolymeren verwendet werden.

Ausgehend von einer verunreinigten Verbindung (A) können durch die vorliegende Erfindung verschiedene hoch-reine Salze der Acrylamido-2-methyl-propansulfonsäure hergestellt werden, die lediglich sehr geringe Mengen an Verunreinigungen, wie z.B. Isobutensulfonsäure (IBSA) und/oder Isobutendisulfonsäure (IBDSA) enthalten.

Der Gehalt dieser Nebenprodukte Isobutensulfonsäure (IBSA) und/oder Isobutendisulfonsäure (IBDSA) soll vorzugsweise insgesamt maximal 100 ppm, insbesondere maximal 70 ppm, vorzugsweise maximal 50 ppm betragen. Gegenstand der Erfindung ist auch ein Natriumsalz der Verbindung (A) mit insgesamt weniger als 100 ppm (IBSA) und (IBDSA).

Dabei kommen in Schritt a) des Verfahrens nicht-wässrige Lösungen eines Alkali-- oder Erdalkalimetalloxids, eines Alkali- oder Erdalkalimetallhydroxids und/oder eines organischen Amins der allgemeinen Formel NR^{a} R^{b} R^{c} zum Einsatz. Die Reste R^{a}, R^{b} und R^{c} bedeuten unabhängig Wasserstoffatome oder Alkyl-, Hydroxyalkyl- oder Alkoxyreste mit 1 bis 4 Kohlenstoffatomen. Beispielsweise kann eine methanolische Lösung von NaOH eingesetzt werden.

Im Allgemeinen stellt die Erfindung ein Verfahren zur Herstellung und Reinigung der Salze der Verbindung (A) dar, bei dem eine nicht-wässrige Lösung oder Suspension eines Oxids, Hydroxids oder eines Amins eingesetzt wird. Bei der Umsetzung der verunreinigten Verbindung (A) mit der basischen Komponente in einem organischen Lösungsmittel entsteht ein Salz.

Die Erfindung betrifft auch ein technisch einfach zu realisierendes Verfahren, um ein hochreines Salz der Verbindung (A), insbesondere mit einer Reinheit von größer als 99 %, insbesondere größer 99,5 %, oftmals größer 99,7 % zu erhalten.

Dieses hochreine Salz eignet sich als Monomer zur Herstellung von hochmolekularen Copolymeren oder Homopolymeren dieses Monomers. Die hergestellten Polymere eignen sich u.a. als Bohrhilfsmittel. Flockungsmittel, Flüssigkeitsverlust-Polymere (fluid loss polymers) und Zementier-Polymere (cementing polymers).

Zur Herstellung der Salze werden z.B. im Wesentlichen organische Lösungen, insbesondere alkoholische Lösungen eines Alkali- oder Erdalkalimetalloxids oder Alkali- oder Erdalkalimetall-hydroxids oder eines Amins verwendet. Die Metallhydroxide der Metalle der IA-Gruppe und IIA-Gruppe des Periodensystems der Elemente sind bevorzugt. Spezielle Beispiele für diese Metalle sind Lithium, Natrium, Kalium, Magnesium und Calcium.

Das besonders bevorzugte Metall der IA-Gruppe ist Natrium und das besonders bevorzugte Metall der IIA-Gruppe ist Magnesium. Von den Metallhydroxid-Lösungen wird bevorzugt eine alkoholische Lösung von Natriumhydroxid verwendet.

Es kann auch eine nicht-wässrige Lösung eines Amins der o. g. allgemeinen Formel NR^{a}R^{b}R^{c} verwendet werden. Es ist auch möglich, eine alkoholische Lösung von Ammoniak zu verwenden.

Der Ausdruck "im Wesentlichen organische Lösung" bedeutet vorliegend, dass das überwiegende Lösungsmittel (L) organisch ist und Wasser nur zu maximal 0,5 Gew.-%, insbesondere zu maximal 0,2 %, oftmals weniger als 0,1 Gew.% im Lösungsmittel enthalten ist.

Als wasserfreie, organische Lösungsmittel sind Alkohole, Aldehyde, Ketone, Nitrile, Ester und Ether mit 1 bis 4 Kohlenstoffatomen, Amide, wie Dimethylformamid oder Sulfoxide, wie Dimethylsulfoxid im Prinzip geeignet. Bevorzugt werden oftmals niedere Alkohole.

Die Umsetzung von Verbindung (A) mit einer Base (B) liefert ein Salz. Es gibt keine technischen Probleme bei der Verwendung eines molaren Überschusses an Base (B). Ein Überschuss von zum Beispiel 1 Mol-% bis 20 Mol-% ist möglich. Um die Salzbildung zu erreichen, kann die basische Komponente (B) mit Verbindung (A) versetzt werden oder umgekehrt. Die Salzbildung ist exotherm und die entstehende Wärme kann dazu verwendet werden, eine maximale Menge Salz im Lösungsmittel zu solubilisieren. Die Salzlösung wird in der Regel anschließend abgekühlt und das feste Salz wird als gereinigtes Produkt zum Beispiel durch Kristallisation gewonnen.

Die Umsetzung von Verbindung (A) mit der Base (B) zu einem Salz erfolgt bei einer Temperatur von 0°C bis 80°C. Vorzugsweise wird die Salzbildung bei einer Temperatur von 5 bis 50°C und insbesondere von 10 bis 40°C durchgeführt.

Das Molverhältnis von Verbindung (A) zu Base (B) bei der Salzbildung ist abhängig von der Beschaffenheit der Base (B). Ist die Base (B) ein Metall der Gruppe IA, so liegt das Molverhältnis von (A):(B) bei 1:1 bis 2, vorzugsweise bei 1:1 bis 1,10 und insbesondere bei 1:1 bis 1,05. Ist die Base (B) ein Metall der Gruppe IIA, so liegt das Molverhältnis von (A): (B) bei 2 : 1 bis 2, vorzugsweise bei 2 : 1 bis 1,10 und insbesondere bei 2 : 1 bis 1,05. Ist die Base (B) ein Amin der allgemeinen Formel NR^{a}R^{b}R^{c}, so ist das Verhältnis der Mole an Verbindung (A) zu den Stickstoffatomen von Base (B) bei 1 : 1 bis 2, vorzugsweise bei 1 : 1 bis 1,10 und insbesondere bei 1 :1 bis 1,05.

Das auf diese Weise gebildete Salz liegt in einer organischen Lösung vor. Die Lösung kann ggf. filtriert werden, um feste Verunreinigungen zu entfernen. Das Salz der Verbindung (A) kann rückgewonnen werden, indem die organische Lösung z. B. Veränderungen in der Temperatur und/oder des Druckes und/oder der Konzentration unterworfen wird.

Auch der Zusatz einer weiteren organischen Komponente (NL), zum Beispiel einer Verbindung, in der das Salz der Verbindung (A) sehr schlecht löslich ist, ist möglich.

Durch Erhöhung der Temperatur wird ggf. bei Unterdruck oftmals ein Teil des Lösungsmittels (L) entfernt, wodurch die Menge an vorliegendem Salz relativ zur Menge an verbleibendem Lösungsmittel ansteigt. Die Entfernung von Lösungsmittel bei erhöhter Temperatur kann durch Verringerung des Druckes vereinfacht werden. Jedoch kann Lösungsmittel (L) auch durch Verringerung des Druckes bei Raumtemperatur entfernt werden. Durch Verringerung der Temperatur wird das Salz aufgrund der Veränderung der Löslichkeit des Salzes in Abhängigkeit von der Temperatur entfernt.

jedem Fall kann das Salz durch Übersättigung der organischen Lösung gewonnen werden. Zwei Verfahren, um eine derartige Übersättigung zu erreichen, sind die Temperaturveränderung und die Kristallisation durch Abstreifen des Lösungsmittels. Beim Verfahren der Temperaturveränderung wird eine gesättigte Lösung abgekühlt, um die Löslichkeit des gewünschten Salzes im Lösungsmittel (L) herabzusetzen. Durch die Herabsetzung der Löslichkeit kristallisiert das Salz aus der Lösung aus. Beim Verfahren der Kristallisation durch Abstreifen des Lösungsmittels wird das Lösungsmittel (L) aus der Lösung entfernt, entweder durch Erwärmen oder durch verminderten Druck oder einer Kombination aus Erwärmen und vermindertem Druck. Die reduzierte Menge an Lösungsmittel bewirkt die Kristallisation des gewünschten Salzes. Die Kristallisation durch Temperaturveränderung und Abstreifen des Lösungsmittels kann diskontinuierlich oder kontinuierlich erfolgen.

Es ist oftmals von Vorteil, dass die organische Lösung bei einer Temperatur von -20° bis etwa 45°C gehalten wird. Temperaturen oberhalb von 45°C können zu einer raschen Bildung von Nebenprodukten oder auch zur Bildung von Polymeren führen. Temperaturen unterhalb -20°C bewirken bisweilen Probleme bei der Isolierung des Salzes aus der organischen Lösung.

Um eine Polymerisation zu verhindern, ist es gegebenenfalls hilfreich, einen Polymerisationsinhibitor zu verwenden. Polymerisationsinhibitoren sind im Handel erhältlich. Ein bevorzugter Polymerisationsinhibitor ist Hydrochinon-monomethylether. Bei einer Verdampfungskristallisation wird das Lösungsmittel unter vermindertem Druck (kleiner 100kPa) abdestilliert, um die Destillationstemperatur zu verringern und um die Nebenproduktbildung möglichst gering zu halten. Ein konstanter Strom eines i Gases, beispielsweise von Spülluft oder eines anderen, Sauerstoff enthaltenden Gases, kann dem Destillations-Vorgang zugeführt werden.

Die Reinheit der bei dem erfindungsgemäßen Verfahren erhaltenen Salze der Verbindung (A) kann beispielsweise durch spektroskopische Methoden, wie beispielsweise H-NMR und C-13 NMR bestimmt werden.

Auch ist eine chromatographische Abtrennung (z.B. HPLC) der in geringen Mengen vorhandenen Nebenprodukte möglich, die dann quantitativ über gängige Methoden bestimmt werden können.

Die nachstehenden Beispiele und Patentansprüche verdeutlichen die Erfindung.

### Beispiel 1

Nachfolgend wird exemplarisch das Herstellungsverfahren von Verbindung (A) in dem Lösungsmittel Acrylnitril in einem üblichen Labor-Maßstab beschrieben, das Verfahren kann aber auch in großem Maßstab durchgeführt werden:
15,5 Mol (820 g) Acrylnitril (AN) werden bei einer Temperatur von -10°C vorgelegt, anschließend werden über zwei Reaktionsmischpumpen 2,5 Mol (140 g) Isobuten (Siedepunkt beträgt -7,1°C), mit einer Geschwindigkeit von 0,6 g/min sowie 2,1 Mol Oleum (205,8 g), mit einer Geschwindigkeit von 22-28 ml/h, gemeinsam zugeführt. Die Zuführung dauert 3,5 h.

Die Temperatur steigt während der Zuführung ständig bis auf 2,7°C an, während mit dem Thermostat (-10°C) gekühlt wird. Nach Beendigung der Zuführung wird auf eine Temperatur von 20°C erwärmt und für einen Zeitraum von 10 min nach gerührt. Es entsteht eine milchige Suspension der Verbindung (A) in dem überschüssigen Lösungsmittel Acrylnitril, wobei auch noch gegebenenfalls Reste von Isobuten und SO₃ vorhanden sein können.

Die Aufreinigung der Verbindung (A) kann durch Kristallisation wie folgt durchgeführt werden:
Die beschriebene milchige Suspension der Verbindung (A) in überschüssigem Lösungsmittel Acrylnitril wird aus dem Reaktionsreaktor abgelassen und in einen zweiten Reaktor eingefüllt. Der Reaktionsreaktor wird nochmals mit 550 ml Essigsäure (96 %) nachgespült und ebenfalls dem zweiten Redaktor zugeführt. Die Reaktionsmischung im zweiten Reaktor wird mit 20 ml Wasser versetzt und unter Rückfluss erwärmt (auf 87°C). Es wird für 10 min unter Rückfluss gerührt und dann abgekühlt. Die ausgefallenen Verbindung (A) wird abgesaugt.

Der Feststoff kann bei 70°C im Trockenschrank über mehrere Stunden getrocknet werden. Man erhält 369,4 g der Verbindung (A), was einer Ausbeute von 85 % entspricht. Das Produkt enthält jedoch noch mehrere Gew.-% an Verunreinigungen.

Bei dem erfindungsgemäßen Verfahren wird hingegen eine verunreinigte Suspension von Verbindung (A) (direkt ohne Aufreinigung oder Abtrennung erhalten aus der Reaktion von Acrylnitril, Isobuten und Oleum) mit einer substantiell wasserfreien Lösung einer Base, bevorzugt Alkalimetall- oder Erdalkalimetallhydroxiden, in einem oder mehreren polaren Lösemitteln (L), wie z.B. Methanol, Ethanol, Isopropanol, Butanole, Acetonitril, Aceton, DMF o.ä. umgesetzt. Vorzugsweise wird ein wasserfreier Alkohol (vorzugsweise Methanol) oder eine Mischung, die überwiegend aus Alkohol (vorzugsweise Methanol) besteht, eingesetzt.

Auch eine Umsetzung mit gasförmigem Ammoniak bzw. Trialkylaminen als Base (B) zu den entsprechenden Ammoniumsalzen ist möglich.

Die erhaltene Lösung oder auch Suspension des Salzes im organischen Lösemittel enthält Verunreinigungen, die durch Reinigungsmethoden, wie beispielsweise Extraktion oder Kristallisation, entfernt werden können. Daher besteht eine bevorzugte Vorgehensweise in der Umkristallisation des erhaltenen Na-Salzes aus einem organischen Lösemittel oder auch einer Mischung zweier oder mehrerer organischer Lösemittel.

Im Gegensatz zur Kristallisation in einem wasserhaltigen System wird dabei das gewünschte Produkt in einer höheren Reinheit (größer 94 %) erhalten. Insbesondere die bei der Polymerisation zu Kettenabbruch und damit zu einem Polymer geringeren Molekulargewichts führenden Verunreinigungen IBSA und IBDSA werden nach dem erfindungsgemäßen Verfahren abgereichert.

Gleiches trifft für eine weitere bevorzugte Vorgehensweise zu, bei der das noch verunreinigte Produkt zunächst filtriert wird und dann mit einem organischen Lösemittel gewaschen wird. Es wird ein Produkt in viel höherer Reinheit erhalten als bei Durchführung der gleichen Vorgehensweise im wässrigen System.

Ein weiterer Vorteil eines Arbeitens im substanziell wasserfreien System besteht darin, dass die Entfernung des Lösemittels beziehungsweise des Lösemittelgemisches deutlich vereinfacht ist. Auch wird das Produkt in einer kristallinen, leicht zu filtrierenden Form erhalten. Es kann einfach getrocknet werden. Im Gegensatz dazu ergibt eine Kristallisation aus wässrigem System ein deutlich schlechter zu filtrierendes Produkt. Auch die Trocknung ist aufwändig.

### Beispiel 2: Herstellung des Natriumsalzes von Verbindung (A) (NaATBS, wasserfrei) direkt aus dem Reaktionsgemisch

Acrylnitril, Isobuten und Oleum wurden wie oben beschrieben miteinander reagiert und es ergab sich eine etwa 25 %ige Suspension von kristallinem ATBS in Acrylnitril.

Diese Lösung wurde bei 5 °C kontrolliert mit einer Lösung von NaOH in trockenem Methanol (18 % NaOH in MeOH) versetzt, bis pH = 7,8 erreicht wurde. Er ergab sich eine homogene, leicht gelbliche Lösung mit einem Gehalt an NaATBS von ca. 16,7 Gew.-% (Bestimmung per geeichter HPLC), die direkt weiter verarbeitet wurde.

### Beispiel 3: Herstellung von NaATBS aus einem substantiell wasserfreien System

Aus 100 g einer nach Beispiel 1 erhaltenen Lösung von NaATBS (16,77 Gew%) in Acrylnitril und Methanol wurde bei Raumtemperatur unter vermindertem Druck und unter Einleitung von Luft in die Reaktionslösung ca. 50 g Lösemittel entfernt. Man erhielt eine Suspension von farblosem, leicht filtrierbarem NaATBS. Das Produkt wurde durch Filtration vom restlichen Lösemittel getrennt und mit weiterem Acrylnitril/Methanol nachgewaschen. Es verblieben 17,3 g eines leicht verunreinigten, feinkristallinem NaATBS (Reinheit 94 %). Der Gewichtsanteil an IBSA lag bei 0,43 %, der Gewichtsanteil an ATB bei 1,7 %.

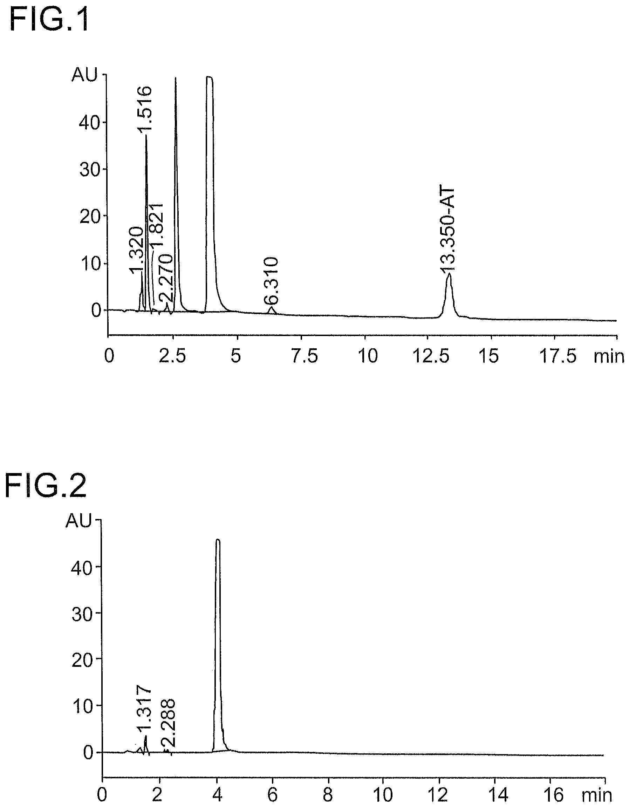

Figur 1 zeigt ein HPLC-Spektrum des erhaltenen Produkts (A). Man erkennt in dem HPLC-Spektrum (Absorption in Abhängigkeit der Zeit (Minuten)), dass neben dem gewünschten Produkt-Peak (bei 4 Minuten) noch zahlreiche unerwünschte Nebenkomponenten wie z.B. tert-Butylacrylamid, Acrylnitril, IBSS und IBDSS vorhanden sind. Somit ist es schwierig, aus der Polymerisation eines solchen Produktes ein Polymer mit ausreichend hohem Molekulargewicht zu erhalten. Die Abszisse des HPLC-Spektrums zeigt die Zeit (0 bis 17,5 Minuten), die Ordinate die Intensität.

### Beispiel 4: Aufreinigung von NaATBS durch Kristallisation aus organischen Lösungsmitteln (substantiell wasserfreie Reaktionsführung)

Aus 809 g einer nach Beispiel 1 erhaltenen Lösung von NaATBS (16,77 Gew.-%) in Acrylnitril und Methanol wurde unter vermindertem Druck und unter Einleitung von Luft in die Reaktionslösung ca. 503 g Destillat entfernt. Zu den verbliebenen 306 g Lösung wurden 383 g Aceton gegeben und die Mischung auf 0°C gekühlt. Nach 15-20 min Dauer begann NaATBS als weiße Kristalle zu fallen. Der erhaltene Niederschlag wurde durch Filtration abgetrennt, mit Aceton gewaschen und vorsichtig bei 70 °C getrocknet. Man erhielt ein aufgereinigtes NaATBS einer Reinheit » 95 %, das kein ATB mehr enthielt und bei dem der Anteil an IBSA auf 0,3 Gew.-% reduziert wurde. Eine wiederholte Anwendung der Methode (Umkristallisation, Waschung) und Trocknen bei 110°C führte zu einem NaATBS hoher Reinheit, das für die Herstellung von Polymeren mit hohem Molekulargewicht geeignet war.

Figur 2 zeigt ein HPLC-Spektrum des erhaltenen Produkts (A). Man erkennt in dem HPLC-Spektrum, dass die unerwünschten Nebenkomponenten deutlich reduziert wurden. Insbesondere wurden die bei der Polymerisation des Monomers (A) störenden Nebenkomponenten IBSS und IBDSS zu < 100 ppm reduziert. Die Abszisse des HPLC-Spektrums zeigt die Zeit (0 bis 16 Minuten), die Ordinate die Intensität.

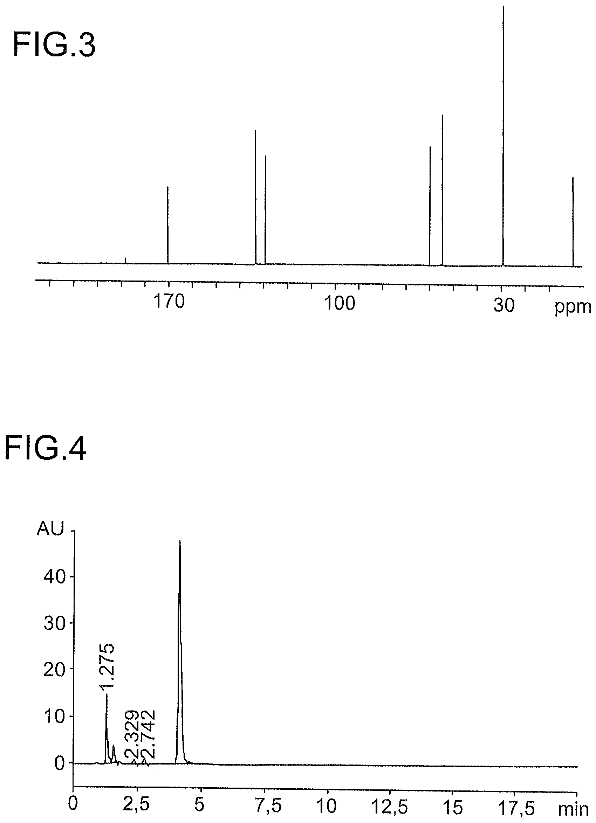

Figur 3 zeigt ein NMR-Spektrum des erhaltenen Produkts (A). Man erkennt in dem NMR-Spektrum (500 MHz; 188,52 ppm; 170,52 ppm (s); 133,70 ppm (d); 129,49 ppm (t); 50,75 ppm (t); 54,72 ppm (s); 29,21 ppm (q), dass das erhaltene Produkt eine sehr hohe Reinheit aufweist. Die Abszisse des NMR-Spektrums zeigt die Verschiebung (220 bis 0 ppm) sowie die Art des Signals (s, d, t oder q). Ein derartig aufgereinigtes Salz der Verbindung (A) ist geeignet zur Herstellung von Polymeren hohen Molekulargewichts.

### Vergleichsbeispiel 1: Kristallisation von Natriumsalz der Verbindung (A) NaATBS aus Wasser (analog US 6,331,647)

Aus 440 g einer 50 %igen Lösung von NaATBS in Wasser wurde unter vermindertem Druck und unter Einleitung von Luft in die Reaktionslösung 100 g Wasser entfernt.

Man erhielt einen zähen, honigartigen Niederschlag, der sich nur schwierig durch Filtration vom Filtrat trennen ließ. Der Filterkuchen wurde unter vermindertem Druck bei 35°C getrocknet. Durch mehrfaches Wiederholen der Prozedur erhielt man in Summe 224 g eines leicht verunreinigten NaATBS (Reinheit 94 %), das nach Trocknen als amorpher Feststoff vorlag. Der Gewichtsanteil an IBSA lag bei 0,43 % - somit ist ein derartig erhaltenes Salz der Verbindung (A) nicht geeignet, um Polymere eines hohen Molekulargewichtes zu erzielen. Das Salz der Verbindung (A) enthielt weiterhin 4-5 Gew.-% Kristallwasser, das sich auch durch Trocknen nicht aus dem Feststoff entfernen ließ.

### Vergleichsbeispiel 2: Aufreinigung von Salz der Verbindung (A) NaATBS durch Kristallisation aus wässriger Lösung mit organischen Lösemitteln

200 g einer etwa 50 %igen Lösung von NaATBS in Wasser (stabilisiert mit 100 ppm MEHQ, Gewichtsanteil an IBSA ca. 0,4 %) wurde bei 20°C unter Rühren mit 400 ml Aceton versetzt.

Es ergab sich ein feiner, weißer Niederschlag, der durch Filtration von der Mutterlauge abgetrennt wurde. Nach vorsichtigem Trocknen bei 70°C wurde der Feststoff per geeichter HPLC analysiert. Es ergab sich eine Reinheit an NaATBS von 84,8 Gew.-% bei einem IBSA-Gewichtsanteil von 3,9 %. Durch diese Verfahrensweise wurde somit die unerwünschte Nebenkomponente IBSA angereichert. Somit ist das erhaltene Produkt (A) (NaATBS) ungeeignet, um Polymere mit hohem Molekulargewicht zu erzielen.

Figur 4 zeigt ein HPLC-Spektrum des erhaltenen Produkts (A). Man erkennt in dem HPLC-Spektrum, dass zahlreiche unerwünschte Nebenkomponenten wie z.B. tert-Butylacrylamid, Acrylnitril, IBSS und IBDSS vorhanden sind. Die Abszisse des HPLC-Spektrums zeigt die Zeit (0 bis 17,5 Minuten), die Ordinate die Intensität. Somit kann aus der Polymerisation eines solchen Produktes kein Polymer mit ausreichend hohem Molekulargewicht erhalten werden.

## Patentansprüche

1. Verfahren zur Herstellung von Salzen von Acrylamido-2-methyl-propansulfonsäure (A) umfassend die Schritte:
a) Herstellen einer Lösung eines verunreinigten Salzes der Acrylamido-2-methyl-propansulfonsäure (A) in einem Wasser-freien, organischen Lösungsmittel (L) unter Einsatz mindestens einer basischen Komponente (B) ausgewählt aus der Gruppe Alkalimetalloxide, Erdalkalimetalloxide, Alkalimetallhydroxide, Erdalkalimetallhydroxide und Amine der allgemeinen Formel (I)
NR^{a} R^{b} R^{c} (I)
wobei die Reste R^{a}, R^{b} und R^{c} unabhängig voneinander bedeuten:
Wasserstoff, C₁-C₄-Alkyl, Hydroxy-C₁-C₄-alkyl oder C₁-C₄-Alkoxy,
wobei das Molverhältnis von Verbindung (A) zur basischen Komponente (B) vorzugsweise bei 1:1 bis 1:3 liegt,
b) gegebenenfalls teilweises Entfernen des organischen Lösungsmittels (L) bei einem Druck im Bereich von 0,1 bis 200 kPa (abs),
c) Rückgewinnung des gelösten Salzes der Verbindung (A) durch Kristallisation oder durch Fällung, durch Veränderung der Temperatur und/oder des Drucks und/oder der Konzentration des Salzes in der Lösung,
d) gegebenenfalls Trocknung des gereinigten Salzes der Acrylamido-2-methyl-propansulfonsäure (A).

2. Verfahren nach Anspruch 1, **dadurch gekennzeichnet, dass** als wasserfreies Lösungsmittel (L) ein Lösungsmittel aus der Gruppe: Methanol, Ethanol, Propanol, Butanol, Acetonitril, Aceton, DMF, oder eine Mischung mindestens zweier dieser Lösungsmittel eingesetzt wird.

3. Verfahren nach Anspruch 1 oder 2, **dadurch gekennzeichnet, dass** in Schritt a) ein Alkalimetall-Salz der Acrylamido-2-methyl-propansulfonsäure, insbesondere das Natrium-Salz eingesetzt wird.

4. Verfahren nach Anspruch 1 oder 2, **dadurch gekennzeichnet, dass** in Schritt a) ein Salz der Acrylamido-2-methyl-propansulfonsäure (A) mit einem Amin der Formel (I), insbesondere ein Trimethylammonium-Salz eingesetzt wird.

5. Verfahren nach einem der Ansprüche 1 bis 4, **dadurch gekennzeichnet, dass** in Schritt b) mindestens 50 Gew.% des organischen Lösungsmittels (L) bei einem Druck im Bereich von 0,1 bis 50 kPa (abs) entfernt werden.

6. Verfahren nach einem der Ansprüche 1 bis 5, **dadurch gekennzeichnet, dass** in Schritt b) mindestens 60 Gew.% des organischen Lösungsmittels (L) unter Einspeisung eines Gases, insbesondere von Luft, entfernt werden.

7. Verfahren nach einem der Ansprüche 1 bis 6, **dadurch gekennzeichnet, dass** in Schritt a) als Lösungsmittel (L) ein wasserfreier C₁-C₃-Alkohol verwendet wird und in Schritt c) das gereinigte Salz durch Temperaturveränderungen aus einer alkoholischen Lösung gewonnen wird.

8. Verfahren nach einem der Ansprüche 1 bis 7, **dadurch gekennzeichnet, dass** in Schritt c) das gereinigte Salz durch Druckveränderungen aus der organischen Lösung gewonnen wird.

9. Verfahren nach einem der Ansprüche 1 bis 8, **dadurch gekennzeichnet, dass** in Schritt c) das gereinigte Salz durch Konzentrationsveränderung und/oder durch Zusatz einer weiteren organischen Komponente (NL) aus der organischen Lösung gewonnen wird.

10. Verfahren nach einem der Ansprüche 1 bis 9, **dadurch gekennzeichnet, dass** mindestens die Schritte a) und c) mehrfach wiederholt werden.

## Claims

1. A process for preparing salts of acrylamido-2-methylpropanesulfonic acid (A) comprising the steps of:
a) preparing a solution of a contaminated salt of acrylamido-2-methylpropanesulfonic acid (A) in an anhydrous organic solvent (L) using at least one basic component (B) selected from the group of alkali metal oxides, alkaline earth metal oxides, alkali metal hydroxides, alkaline earth metal hydroxides and amines of the general formula (I)
NR^{a} R^{b} R^{c} (I)
where the R^{a}, R^{b} and R^{c} radicals are each independently:
hydrogen, C₁-C₄-alkyl, hydroxy-C₁-C₄-alkyl or C₁-C₄-alkoxy,
where the molar ratio of compound (A) to the basic component (B) is preferably 1:1 to 1:3,
b) optionally partly removing the organic solvent (L) at a pressure in the range from 0.1 to 200 kPa (abs),
c) recovering the dissolved salt of compound (A) by crystallization or by precipitation, by altering the temperature and/or the pressure and/or the concentration of the salt in the solution,
d) optionally drying the purified salt of acrylamido-2-methylpropanesulfonic acid (A).

2. The process according to claim 1, wherein the anhydrous solvent (L) used is a solvent from the group of: methanol, ethanol, propanol, butanol, acetonitrile, acetone, DMF, or a mixture of at least two of these solvents.

3. The process according to claim 1 or 2, wherein, in step a), an alkali metal salt of acrylamido-2-methylpropanesulfonic acid, especially the sodium salt, is used.

4. The process according to claim 1 or 2, wherein, in step a), a salt of acrylamido-2-methylpropanesulfonic acid (A) with an amine of the formula (I), especially a trimethylammonium salt, is used.

5. The process according to any of claims 1 to 4, wherein, in step b), at least 50% by weight of the organic solvent (L) is removed at a pressure in the range from 0.1 to 50 kPa (abs).

6. The process according to any of claims 1 to 5, wherein, in step b), at least 60% by weight of the organic solvent (L) is removed while feeding in a gas, especially air.

7. The process according to any of claims 1 to 6, wherein, in step a), the solvent (L) used is an anhydrous C₁-C₃-alcohol, and, in step c), the purified salt is obtained from an alcoholic solution by temperature changes.

8. The process according to any of claims 1 to 7, wherein, in step c), the purified salt is obtained from the organic solution by pressure changes.

9. The process according to any of claims 1 to 8, wherein, in step c), the purified salt is obtained from the organic solution by changing the concentration and/or by adding a further organic component (NL).

10. The process according to any of claims 1 to 9, wherein at least steps a) and c) are repeated more than once.

## Revendications

1. Procédé de fabrication de sels de l'acide acrylamido-2-méthyl-propanesulfonique (A), comprenant les étapes suivantes :
a) la fabrication d'une solution d'un sel de l'acide acrylamido-2-méthyl-propanesulfonique (A) contaminé dans un solvant organique anhydre (L) en utilisant au moins un composant basique (B) choisi dans le groupe constitué par les oxydes de métaux alcalins, les oxydes de métaux alcalino-terreux, les hydroxydes de métaux alcalins, les hydroxydes de métaux alcalino-terreux et les amines de formule générale (I)
NR^{a}R^{b}R^{c} (I)
dans laquelle les radicaux R^{a}, R^{b} et R^{c} signifient indépendamment les uns des autres : hydrogène, alkyle en C₁-C₄, hydroxy-alkyle en C₁-C₄ ou alcoxy en C₁-C₄,
le rapport molaire entre le composé (A) et le composant basique (B) étant de préférence de 1:1 à 1:3,
b) éventuellement l'élimination partielle du solvant organique (L) à une pression dans la plage allant de 0,1 à 200 kPa (abs),
c) la récupération du sel du composé (A) dissous par cristallisation ou par précipitation, par modification de la température et/ou de la pression et/ou de la concentration du sel dans la solution,
d) éventuellement le séchage du sel de l'acide acrylamido-2-méthyl-propanesulfonique (A) purifié.

2. Procédé selon la revendication 1, **caractérisé en ce qu'**un solvant du groupe constitué par : le méthanol, l'éthanol, le propanol, le butanol, l'acétonitrile, l'acétone, le DMF ou un mélange d'au moins deux de ces solvants est utilisé en tant que solvant anhydre (L).

3. Procédé selon la revendication 1 ou 2, **caractérisé en ce qu'**un sel de métal alcalin de l'acide acrylamido-2-méthyl-propanesulfonique, notamment le sel de sodium, est utilisé à l'étape a).

4. Procédé selon la revendication 1 ou 2, **caractérisé en ce qu'**un sel de l'acide acrylamido-2-méthyl-propanesulfonique (A) avec une amine de formule (I), notamment un sel de triméthylammonium, est utilisé à l'étape a).

5. Procédé selon l'une quelconque des revendications 1 à 4, **caractérisé en ce qu'**au moins 50 % en poids du solvant organique (L) est éliminé à une pression dans la plage allant de 0,1 à 50 kPa (abs) à l'étape b).

6. Procédé selon l'une quelconque des revendications 1 à 5, **caractérisé en ce qu'**au moins 60 % en poids du solvant organique (L) est éliminé à l'étape b) par introduction d'un gaz, notamment d'air.

7. Procédé selon l'une quelconque des revendications 1 à 6, **caractérisé en ce qu'**un alcool en C₁-C₃ anhydre est utilisé en tant que solvant (L) à l'étape a) et le sel purifié est obtenu à partir d'une solution alcoolique par des modifications de température à l'étape c).

8. Procédé selon l'une quelconque des revendications 1 à 7, **caractérisé en ce que** le sel purifié est obtenu à partir de la solution organique par des modifications de pression à l'étape c).

9. Procédé selon l'une quelconque des revendications 1 à 8, **caractérisé en ce que** le sel purifié est obtenu à partir de la solution organique par une modification de concentration et/ou par ajout d'un composant organique supplémentaire (NL) à l' étape c).

10. Procédé selon l'une quelconque des revendications 1 à 9, **caractérisé en ce qu'**au moins les étapes a) à c) sont répétées à plusieurs reprises.
